# EUROPEAN PATENT APPLICATION

(11) **EP 4 767 967 A2**
(43) Date of publication of application: **01.07.2026**
(21) Application number: 25220680.0
(22) Date of filing: 04.12.2025
(51) Int. Cl.: A61B 18/14, A61B 5/0538, H05K 1/02, A61B 5/318, A61B 5/287

(54) **REINFORCED FLEXIBLE CIRCUIT FOR AN END EFFECTOR OF A MEDICAL CATHETER**

(30) Priority: 31.12.2024 US 202419007378
(71) Applicant: Biosense Webster (Israel) Ltd., 2066717 Yokneam (IL)
(72) Inventor: EBRAHIMI, Babak, Irvine, 92618 (US); ABBAS, Mohammad, Irvine, 92618 (US); RODRIGUEZ SOTO, Juan, Irvine, 92618 (US); VAN NIEKERK, Pieter Emmelius, Irvine, 92618 (US)
(74) Representative: Carpmaels & Ransford LLP

(57) **Abstract**

The disclosed technology includes an end effector comprising at least one flexible circuit extending along a longitudinal axis having a substrate supporting at least one electrical trace and at least one electrode. Portions of the substrate may be reinforced by forming the electrical trace with a plurality of bends and/or by providing a plurality of bends in the substrate. Additionally, portions of the substrate may include multiple layers, each layer having electrical traces which are offset from one another.

## Description

### FIELD

The present invention relates generally to minimally invasive medical devices, and in particular sensing catheters, and further relates to, but not exclusively, cardiac mapping catheters and the manufacture thereof.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

It may be desirable for such a catheter to be capable of flexing to allow sufficient electrode contact with different tissue surfaces, for example, flat, curved, irregular or nonplanar surface tissue, and be collapsible for atraumatic advancement and withdrawal through a patient's vasculature. As will be appreciated, flexible circuits used in ablation catheters are often limited in the amount of flexure they are able to take without breaking. Thus, it is desirable to develop new ways to ensure the flexible circuits used in the end effectors of catheters are capable of sufficiently flexing without breaking. These and other advantages are disclosed herein.

### SUMMARY

There is provided, in accordance with the disclosed technology, a flexible circuit of an end effector of a medical probe. The end effector may extend along a longitudinal axis. The flexible circuit may include a first substrate supporting a first electrical trace extending along a portion of the end effector. The first electrical trace may include a plurality of bends such that the first electrical trace defines a serpentine shape. A second substrate may be provided and may be layered with the first substrate in a thickness direction. The second substrate may support a second electrical trace extending along the portion of the end effector which the first electrical trace also extends along. The second electrical trace may also comprise a plurality of bends such that the second electrical trace also defines a serpentine shape. The second electrical trace may offset from the first electrical trace at one or more bends of the plurality of bends.

The disclosed technology may include an end effector comprising a flexible circuit comprising a plurality of electrodes. The flexible circuit may be disposed within a first insulative layer of an insulative material and each electrode of the plurality of electrodes may include a contact surface. The insulative material may be contiguous to the contact surface so that only the contact surfaces of at least a portion of the plurality of electrodes are exposed to an ambient environment. A framework may be at least partially encapsulated in the first insulative layer and a lumen may be defined within the insulative material.

The disclosed technology may include a medical probe including an end effector. The end effector may extend along a longitudinal and include a flexible circuit. The flexible circuit may include a substrate supporting an electrical trace, the electrical trace extending along a portion of the end effector. The substrate of the flexible circuit may comprise a plurality of bends such that the substrate defines a serpentine shape. the plurality of bends of the substrate may be offset relative to the longitudinal axis by an offset angle The electrical trace may be spaced a predetermined distance from an inner radius of each bend.

The disclosed technology may include a medical probe including an end effector. The end effector may extend along a longitudinal and include a flexible circuit. The flexible circuit may include a substrate, the substrate having a proximal portion extending distally from a proximal end of the flexible circuit toward a first set of electrodes of a plurality of electrodes. The substrate may support a first electrical trace comprising a plurality of bends such that the electrical trace comprises a serpentine shape. A middle portion of the substrate, extending between the first set of electrodes and a second set of electrodes may include a plurality of bends such that the substrate defines a serpentine shape in the middle portion. The bends may be offset relative to the longitudinal axis by an offset angle.

Additional features, functionalities, and applications of the disclosed technology are discussed in more detail herein.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
FIG. 1 is a schematic pictorial illustration of a medical system including a medical device, in accordance with the disclosed technology;
FIG. 2 is a schematic pictorial illustration of a perspective view of an end effector, in accordance with the disclosed technology;
FIG. 3 is a schematic pictorial illustration of an exploded perspective view of the end effector of FIG. 2, in accordance with the disclosed technology;
FIG. 4 is a schematic pictorial illustration of a flexible circuit of an end effector, in accordance with the disclosed technology;
FIG. 5 is a schematic pictorial illustration of a substrate of a flexible circuit of an end effector having one or more electrical traces provided thereon, in accordance with the disclosed technology;
FIG. 6A is a schematic pictorial illustration of a distal corner of a flexible circuit of an end effector, in accordance with the disclosed technology;
FIG. 6B is a schematic pictorial illustration of a first substrate of a flexible circuit at a distal corner of an end effector, in accordance with the disclosed technology;
FIG. 6C is a schematic pictorial illustration of a detailed view of FIG. 6B showing the first substrate at a distal corner of flexible circuit of an end effector, in accordance with the disclosed technology;
FIG. 6D is a schematic pictorial illustration of a second substrate of a flexible circuit at a distal corner of an end effector, in accordance with the disclosed technology;
FIG. 6E is a schematic pictorial illustration of a detailed view of FIG. 6D showing the second substrate of a flexible circuit at a distal corner of an end effector, in accordance with the disclosed technology;
FIG. 7 is a schematic pictorial illustration of a detailed view of a middle portion of a flexible circuit of an end effector, in accordance with the disclosed technology;
FIG. 8A is a finite element analysis visualization depicting strain concentrations on a substrate of a flexible circuit of an end effector under tension, in accordance with the disclosed technology;
FIG. 8B is a finite element analysis visualization depicting strain concentrations on a substrate of a flexible circuit of an end effector under tension, in accordance with the disclosed technology;
FIG. 8C is a finite element analysis visualization depicting strain concentrations on a substrate of a flexible circuit of an end effector under tension, in accordance with the disclosed technology;
FIG. 8D is a finite element analysis visualization depicting strain concentrations on a substrate of a flexible circuit of an end effector under tension, in accordance with the disclosed technology;
FIG. 8E is a finite element analysis visualization depicting strain concentrations on a substrate of a flexible circuit of an end effector under tension, in accordance with the disclosed technology;
FIG. 8F is a graphical representation of depicting strain and stiffness a substrate of a flexible circuit of an end effector under tension in various configurations, in accordance with the disclosed technology; and
FIG. 9 is a schematic pictorial illustration of a detailed view of a proximal portion of a flexible circuit of an end effector, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, reference to tissue, vasculature, or organs of a "patient," "host," "user," and "subject" can be that of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator or any other individual or delivery instrumentation associated with delivery of a multi-electrode medical device for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells. For example, by utilizing thermal energy, such as radio frequency (RF) ablation, or non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, methods or uses and devices for mapping and ablation of cardiac tissue to treat cardiac arrhythmias. Ablative energies are typically provided to cardiac tissue by a tip portion of a medical device (e.g., a medical probe or catheter) which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

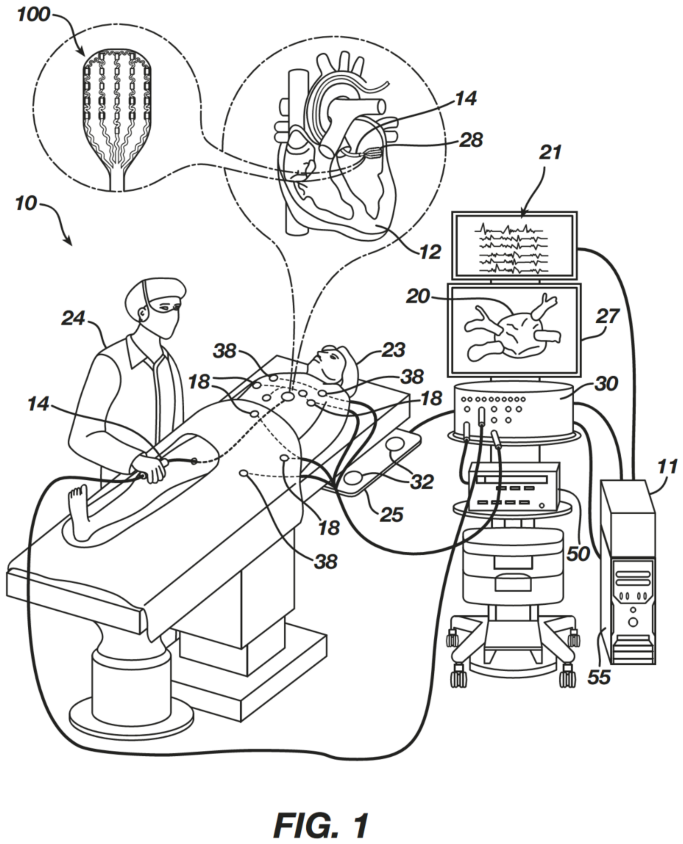

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a catheter shaft with distal tip 28 of catheter 14 (i.e., multilayered end effector 100) into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes optionally distributed over end effector 100 coupled to a catheter shaft and configured to sense the IEGM signals as described in more detail below. Catheter 14 may additionally include a position sensor embedded in or near end effector 100 for tracking position and orientation of end effector 100. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of end effector 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes. For impedance-based tracking, electrical current is directed toward the electrodes and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes of the catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip 28 of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

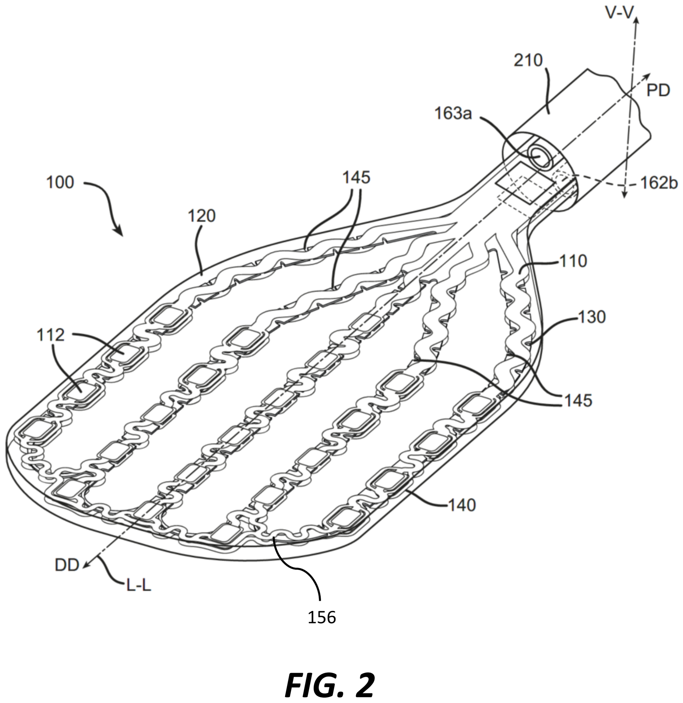

FIG. 2 illustrates an end effector 100 in accordance with an example of the present disclosure in order to achieve the ease of manufacturing, reduced cost, and enhanced end effector properties, such as desired collapsibility, maneuverability, robustness, stiffness, mapping resolution, electrode contact with target anatomy, and conformity of the end effector 100 disclosed herein to flat, curved, irregular and/or nonplanar tissue surfaces found in the target anatomy. The end effector 100 may extend along a longitudinal axis L-L from a proximal end to a distal end, a width extending perpendicular to the longitudinal axis, and a thickness extending in a direction orthogonal to the longitudinal axis and the horizontal axis.

The end effector 100 can include a flexible circuit 110 including a plurality of electrodes As used herein, the term "flexible circuit" includes thin-film circuit, flexible printed circuit board, thin film deposition via lithography and etching processes on substrate such as polyimide, copper, LCP (e.g., Panasonic FELIOS^{™} SERIES of Flexible Circuit Boards), nitinol substrate or directly onto the polymeric substrate such as Panasonic BEYOLEX^{™} PRINTED ELECTRONICS SUBSTRATE Series. In some examples, the flexible circuits described herein can be made primarily of polyimide. In other examples, it can be made of any of biocompatible polyimides, glass-reinforced epoxy laminate materials, copper, or graphene, alone or in combination. The first 110 and second 140 flexible circuits can include conductive traces directly formed onto the polymeric substrate with the electrode comprising a substantially much thicker version of the traces such that the typical polyimide substrate is no longer required. In some examples, the electrodes described herein can include at least one mapping electrode and/or at least one ablation electrode and can be configured to detect electrophysiological signals or transmit ablative energy AC or DC from an energy generator to the tissue according to the various ablation methods previously described e.g., RF, IRE, etc.

The flexible circuit 110 can be disposed on an insulative material 120 (also known as an insulative mass). The insulative material 120 can be contiguous to the contact surfaces of the electrodes so that only the contact surfaces of at least a portion of the plurality of electrodes 112 are exposed to the ambient environment. As used herein, the term "contact surface" includes the portion of an electrode having a generally flat surface and the edge or edges which immediately surround said flat surface. Electrodes 112 may have a slightly rounded, radiused, or chamfered edge that comes into contact with tissue, along with the generally flat surface, when the end effector 100 is placed against tissue. As used herein, "ambient environment" refers to the external environment such as the organ in which the end effector 100 is deployed or in the operating theater prior to being deployed in the biological organ.

It is noted that not all of the electrodes 112 on the end effector 100 described herein need be exposed through the insulative material 120 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood. Irrigation can be provided with irrigation ports 163a on one side and 162b on the other side in fluid communication with an irrigation line not shown disposed in a catheter shaft 210. Instead of an irrigation line separate from the catheter shaft 210, a lumen can be formed via extrusion of the catheter shaft 210 to provide for a lumen channel. It is noted that port 163a or 162b can be configured to have a sufficient flow diverter characteristic for irrigation fluid to cover the mapping electrodes during irrigation flow so as to prevent or reduce thrombus formations.

The end effector 100 can further include a framework 130 contiguous to the insulative material 120 or at least partially encapsulated in the insulative material 120. In examples in which the end effector 100 includes framework 130, the framework 130 can be spaced from the flexible circuit 110 by some of the insulative material 120 coming therebetween.

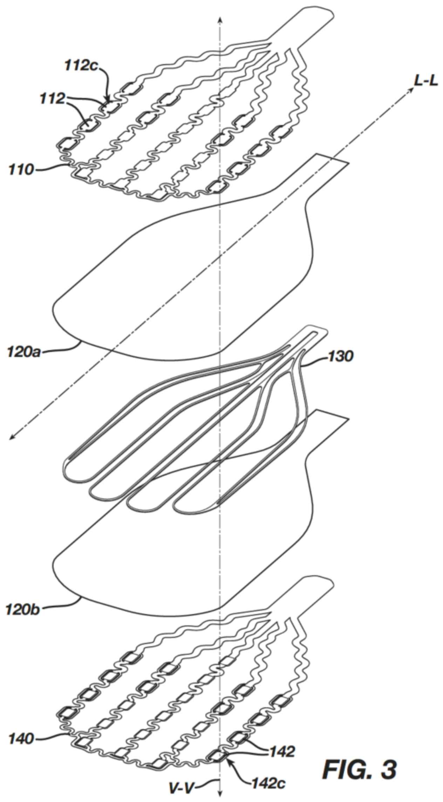

FIG. 3 shows an exploded view of the end effector 100, with the components thereof exploded vertically along vertical axis V-V (also referred to as the height direction) of the end effector 100. The flexible circuit 110 can be a first flexible circuit 110 and the plurality of electrodes 112 can be a plurality of first electrodes 112. The end effector 100 can further include a second flexible circuit 140 having a plurality of second electrodes 142. The second flexible circuit 140 can be spaced apart from the first flexible circuit 110. Moreover, each flexible circuit 110, 140 may include respective tines extending along the longitudinal axis L-L, with the tines supporting the respective electrodes.

In examples having first 110 and second 140 flexible circuits, the insulative material 120 can be disposed between the first flexible circuit 110 and the second flexible circuit 140. In some examples, the insulative material 120 can be contiguous to the contact surfaces of the electrodes so that only the contact surface of each second electrode is exposed to the ambient environment.

Electrodes 112, 142 can sense electrophysiological signals or transmit ablative energy from an energy generator to tissue. In some examples, there are about 92 electrodes. In some examples, there are about 48 electrodes. In some examples, there are about 64 electrodes. In some examples, there are about 72 electrodes. In some examples, there are about 98 electrodes. However, those skilled in the art will appreciate that various numbers of electrodes 112, 142 can be employed without departing from the spirit and scope of the present disclosure.

In examples herein, the end effector 100 includes a framework 130 disposed between the first flexible circuit 110 and the second flexible circuit 140. Framework 130 can be a component of the end effector 100 that is separate and distinct from the first flexible circuit 110 and disposed proximate the first flexible circuit 110. In this case, the insulative material 120 can be further disposed between the framework 130 and the second flexible circuit 140. The framework 130 can be formed from a planar or cylindrical stock of material using any suitable method. For example, the framework 130 can be formed by cutting, laser cutting, stamping, etc.

The framework includes a plurality of spines extending along the longitudinal axis L-L. The tines of the flexible circuits 110, 140 can be approximately aligned with one or more of the spines such that the spines 132 provide support thereto.

Insulative material 120 can include a first sheet of insulative material and a second sheet of insulative material fused together proximate the framework 130 into a single, contiguous, generally planar insulative material 120. This insulative material 120 also serves to enhance the atraumaticity of the end effector 100 and to protect the subject from sharp edges.

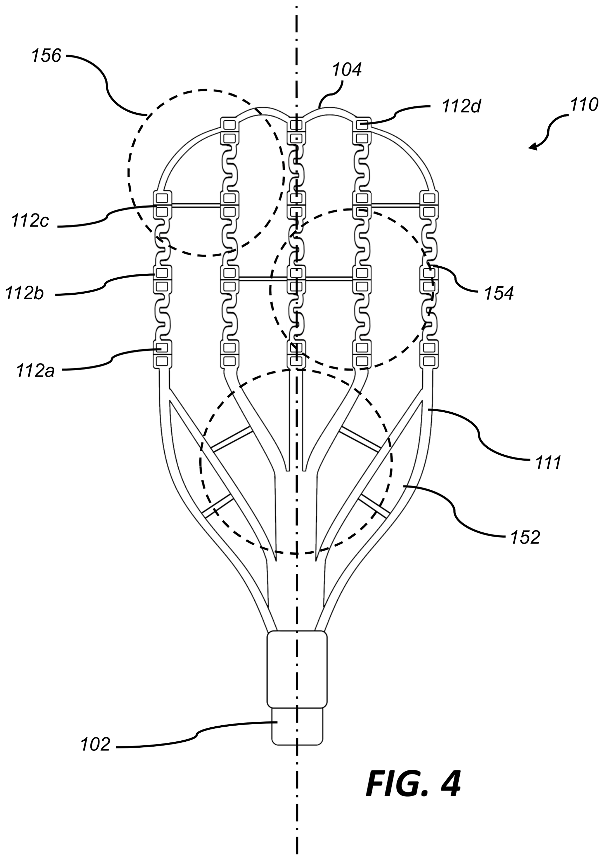

With reference to FIG. 4, a flexible circuit 110 of an end effector is depicted extending along a longitudinal axis L-L. As discussed above, an end effector may comprise a first flexible circuit 110 and a second flexible circuit. For simplicity, a single flexible circuit may be referred to as a flexible circuit 110 herein, but is should be appreciated that the examples and features of the flexible circuit discussed below can be applied to both a first flexible circuit and a second flexible circuit, and further flexible circuit of an end effector.

In some examples, with reference to FIG. 4, the flexible circuit may extend from a proximal end 102 to a distal end 104 along a longitudinal axis. The flexible circuit 110 may comprise a substrate 111 configured to support one or more electrodes. In some examples, the flexible circuit comprises one or more rows or sets of electrodes arranged along the longitudinal axis. Electrical traces (not shown in FIG. 4) may connect the electrodes to the proximal end 102 of the flexible circuit and/or to one another.

In some examples, as depicted in FIG. 4, the flexible circuit 110 may comprise a proximal portion 152 extending between the proximal end 102 of the flexible circuit to a first set of electrodes 112a. In some examples, the flexible circuit 110 comprises a middle portion 154 extending between the first set of electrodes 112a and a third set of electrodes 112c, and may include a second set of electrodes 112b. A fourth set of electrodes 112d may be provided at a distal end 104 of the flexible circuit. In some examples, the first set of electrodes 112a, the second set of electrodes 112b, and the third set of electrodes 112c each comprise an equal number of electrode pairs. For example, as depicted in FIG. 4 the first, second, and third sets of electrodes 112a, 112b, 112c each comprise five electrode pairs or 10 individual electrodes each. In some examples, the fourth set of electrodes 112d, comprises less electrodes than the first, second, and third sets of electrodes 112a, 112b, 112c. For example, as depicted in FIG. 4, the fourth set of electrodes 112d may only comprise 3 pairs of electrodes. A distal corner 156 of the flexible circuit 110 may comprise a curved portion of the substrate 111 extending from the outermost electrode pairs of the third electrode set 112c to the outermost electrode pairs of the fourth electrode set 112d.

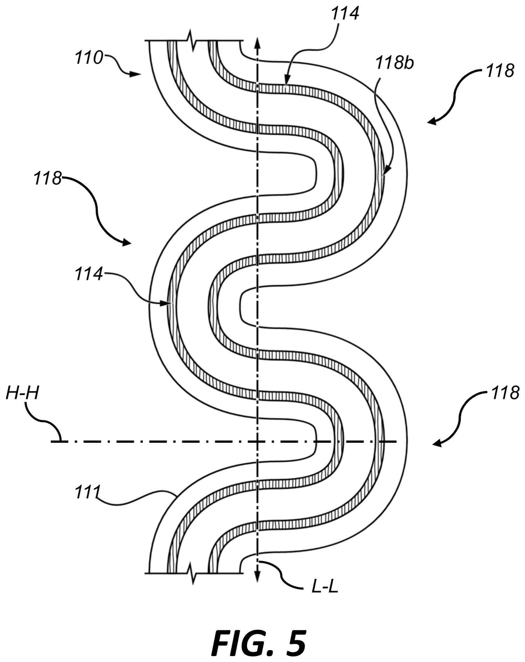

With reference to FIG. 5, a portion of a flexible circuit 110 of an end effector is depicted extending along a longitudinal axis L-L. In some examples, the substrate 111 comprises a plurality of bends 118, such that the substrate 111 comprises a serpentine shape. In some examples, the substrate supports one or more electrical traces 114. In some examples, as depicted in FIG. 5, electrical traces comprise a plurality of bends 118b such that the electrical traces 114 follow the curvature of the substrate 111 and form a serpentine shape. In some examples, each bend 118 is substantially U-shaped, such that the substrate, and the electrical traces 114 formed thereon, turn 180 degrees through the bend.

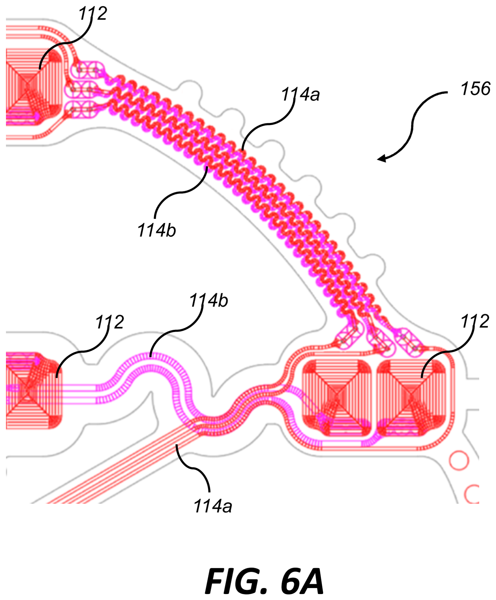

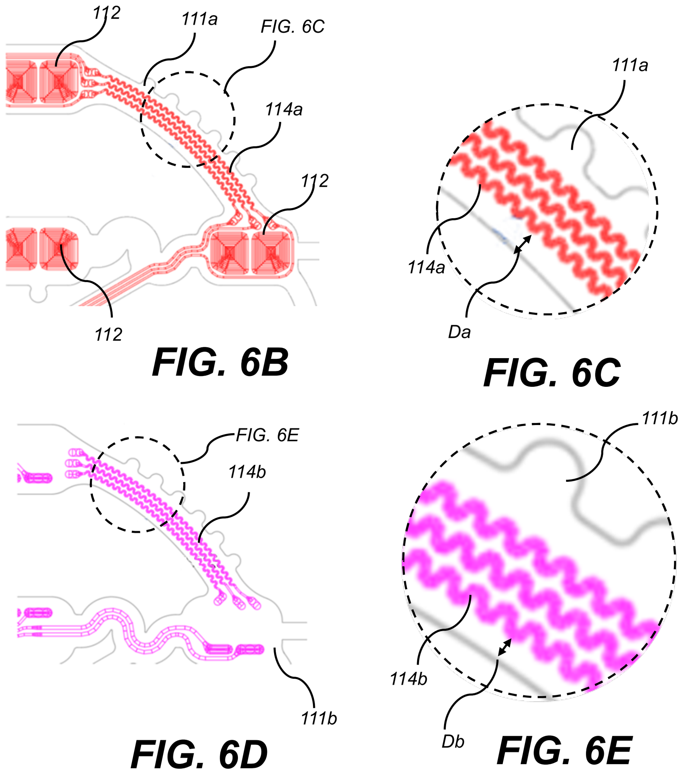

With reference to FIG. 6A-6E, the flexible circuit 110 comprises a first substrate 111a and a second substrate 111b layered with the first substrate 111a in a thickness along at least a portion of the flexible circuit. In some examples, at least one first electrical trace 114a is provided on the first substrate 111a, and at least one second electrical trace 114b is provided on the second substrate 111b. In some examples, the second electrical trace 114b is offset from the first electrical trace 114a to provide reinforcement over at portion of the flexible circuit.

FIG. 6A depicts a distal corner 156 of a flexible circuit wherein the first substrate is layered on to the second substrate, such that first electrical traces 114a and second electrical traces 114b are provided between electrodes 112. FIG. 6B depicts only the first substrate 111a, having first electrical traces 114a provided thereon. FIG. 6C is a detailed view of FIG. 6C illustrating the distance Dₐ between the innermost edge of the first substrate 111a and an innermost edge of the first electrical traces 114a. FIG. 6D depicts only the second substrate 111b, having second electrical traces 114b provided thereon. FIG. 6F is a detailed view of FIG. 6D illustrating the distance D_{b} between the innermost edge of the second substrate 111b and an innermost edge of the second electrical traces 114b.

In some examples, the first electrical traces 114a comprise a plurality of bends such that the first electrical traces 114a form a serpentine shape. The second electrical traces 114b may also comprise a plurality of bends such that the second electrical traces 114b define a serpentine shape. The second electrical traces 114b and the first electrical trace 114a, each having a serpentine shape with a plurality of bends, may also be offset from one another to provide reinforcement over at portion of the flexible circuit.

In some examples, the configuration of layered substrates with offset electrical traces may be utilized to reinforce a distal corner 156 of the flexible circuit. However, it will be appreciated that such a layered configuration with offset electrical traces can be applied to other areas of the flexible circuit.

In some examples, a width of the first substrate 111a is equal to a width of the second substrate 111b along the portion of the flexible circuit with a layered substrate configuration. In some examples, the first electrical trace 114a is disposed at a first distance Dₐ from an edge of the first substrate 111a, and the second electrical trace 114b is disposed at a second distance D_{b} from a corresponding edge of the second substrate 111b. In some examples, the distance Dₐ from an edge of the first substrate 111a to an edge of the first electrical traces 114a is less than or greater than the second distance D_{b} from an edge of the second substrate to an edge of the second electrical traces 114b, such that when first substrate 111a and second substrate 111b are aligned and layered, the first electrical trace 114a and the second electrical trace 114b are offset from one another. In some examples, the distance Dₐ from an edge of the first substrate 111a to an edge of the first electrical traces 114a is less than or greater than the second distance D_{b} by a distance equivalent to the width of the first electrical trace 114a or the second electrical trace 114b. For example, if the widths of the first electrical trace 114a and the second electrical trace 114b are 25 microns, and the first electrical trace 114a is disposed at a distance Dₐ of 75 microns from the edge of the first substrate, then second electrical trace 114b may be disposed a distance D_{b} of 50 microns from the edge of the second substrate.

In some examples, electrodes 112 are formed on the first substrate 111a. In some examples, electrodes 112 are formed on the second substrate. The first electrical traces 114a and the second electrical traces 1114b may extend between and couple adjacent electrodes to one other. By providing two sets of electrical traces, an electrical connection may be maintained even if one of the electrical traces fails or becomes damaged.

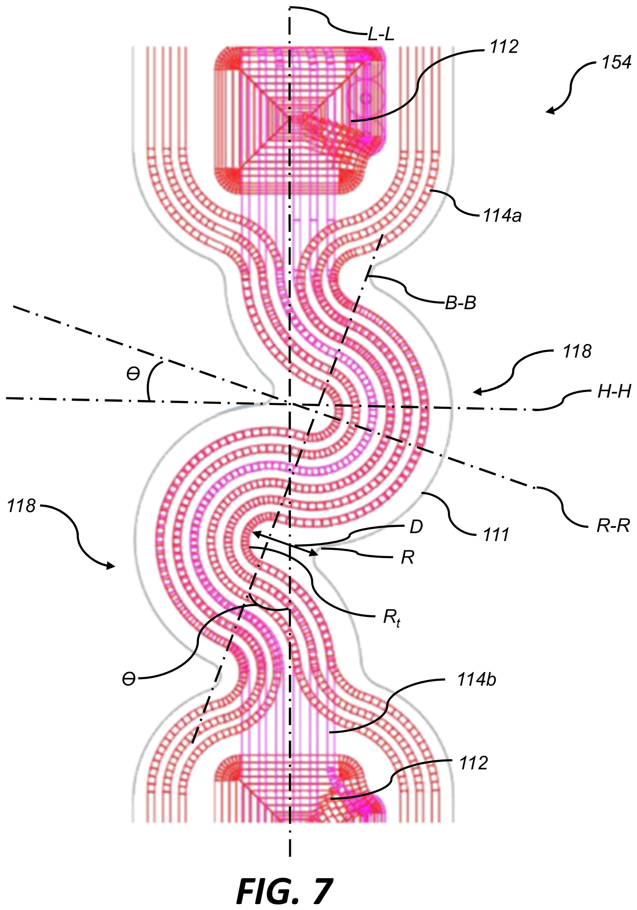

FIG. 7 depicts a portion of a middle portion 154 of a flexible circuit where two electrodes, which are aligned along or parallel to the longitudinal axis L-L are connected by a plurality of electrical traces 114a, 114b provided on a substrate 111. As discussed above, substrate 111 may also be a multilayered substrate having a first set of electrical traces 114a disposed on a first substrate and a second set of electrical traces 114b disposed on a second substrate.

In some examples, the substrate 111 comprises a plurality of bends 118. In some examples, each bend 118 is substantially U-shaped, such that the substrate, and the electrical traces 114 formed thereon, turn 180 degrees through the bend. In some examples, as depicted in FIG. 7, electrical traces 114 follow the curvature of the substrate 111 and also form a serpentine shape. In some examples, a portion of the substrate 111 provided between two electrodes 112 in a middle portion 154 of the flexible circuit comprises two bends 118.

In some examples, the electrical traces 114 are provided a predetermined distance D between the inner radius R of each bend 118. In some examples, the predetermined distance D is measured between an innermost edge of the substrate 111 at the inner radius R of each bend 118 and an innermost electrical trace 114 closest to said innermost edge. In some examples, as substrate 111 is flexed, stress and strain concentrate at the inner radius of each bend 118. By offsetting the innermost electrical trace 114 by a distance D away from the inner radius of the substrate 111, the electrical traces 114 will be subjected to less stress/strain. In some examples, the predetermined distance D is equal to or greater than the 3 times the radius Rt of the inner most electrical trace 114. For example, if the innermost electrical trace 114 has a radius Rₜ of 0.5mm, the innermost electrical trace 114 should be provided at least 1.5 mm away from the inner radius R of each bend.

In some examples, each bend 118 of the substrate 111 comprises a radius of curvature R. The radius of curvature R may be measured at the innermost curve of the substrate 111 at each bend 118. In some examples, the inner radius R of each bend 118 is at least approximately 0.01 millimeters. In some examples, the inner radius R of each bend 118 is at least approximately 0.03 millimeters. In some examples, the inner radius R of each bend 118 is from approximately 0.03mm to approximately 0.07mm.

In some examples, as depicted in FIG. 7, the bends 118 are offset by an angle Θ. The offset angle Θ may be measured relative to the longitudinal axis L-L, or an axis parallel thereto which extends from through the center of electrodes 112 in a middle portion of the flexible circuit. For example, as depicted in FIG. 7, if electrodes 112 are provided along the longitudinal axis L-L, offset angle Θ may be measured between the longitudinal axis L-L and an axis B-B which passes through the center of bends 118. The offset angle Θ may also be measured between a horizontal axis H-H, or an axis perpendicular to the longitudinal axis L-L, and an axis R-R which bisects the radius R of each bend 118 of the substrate 111. In comparison, the substrate 111 depicted in FIG. 5 is not offset, and the bends 118 run along the longitudinal axis L-L, or along an axis parallel to the longitudinal axis L-L, and the horizontal axis H-H, or an axis parallel thereto or perpendicular with the longitudinal axis L-L, bisects the radius of curvature at each bend 118.

In some examples, the offset angle Θ is approximately 10 degrees to 35 degrees. In some examples, the , the offset angle Θ is approximately 10 degrees to 20 degrees.

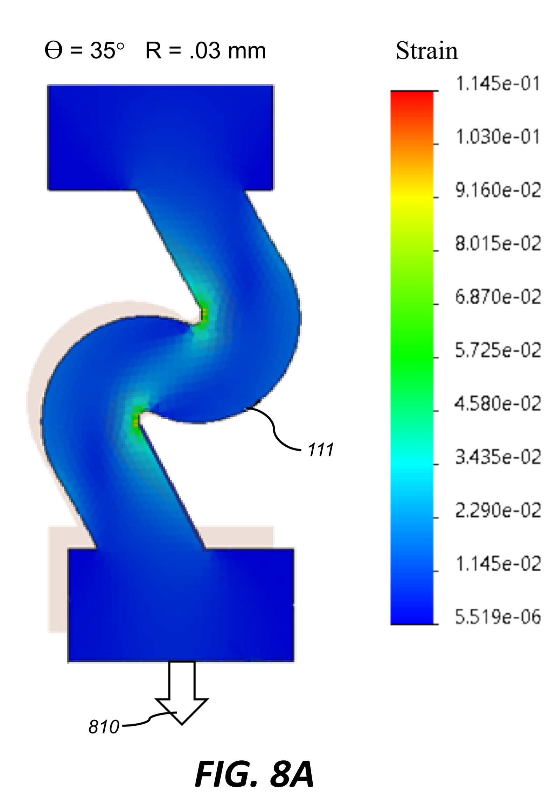

In some examples, providing on offset angle Θ may decrease the strain experienced through the bends 118 of the substrate 111 during use. In some examples, offset angle Θ may decrease the stiffness of the substrate. FIGS. 8A-8E depict a finite element analysis conducted on various configurations of a substrate 111 being elongated by 0.1 millimeters (mm) in a direction 810, showing the exemplary configuration with strain mapped onto the substrate 111 and a corresponding color bar representing the strain values mapped onto the substrate. As depicted in FIGS. 8A-8E, the maximum strain is experienced at the inner radius of the substrate 111 at each bend.

FIG. 8A depicts a substrate 111 having an offset angle Θ of 35 degrees and a radius R of 0.03 mm. In the exemplary configuration, when elongated by 0.1 mm, the substrate experiences a maximum strain of 0.1145 or about 11.45%.

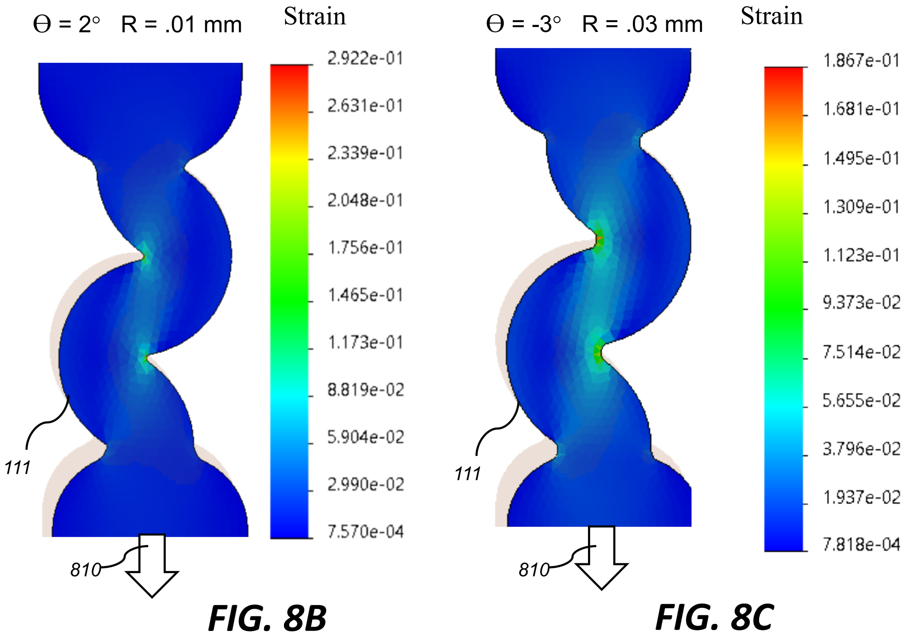

FIG. 8B depicts a substrate 111 having an offset angle Θ of 2 degrees and a radius R of 0.01 mm. In the exemplary configuration, when elongated by 0.1mm, the substrate experiences a maximum strain of 0.2922 or about 29.22%.

FIG. 8C depicts a substrate 111 having an offset angle Θ of -3 degrees and a radius R of 0.03 mm. In the exemplary configuration, when elongated by 0.1mm, the substrate experiences a maximum strain of 0.1867 or about 18.67%.

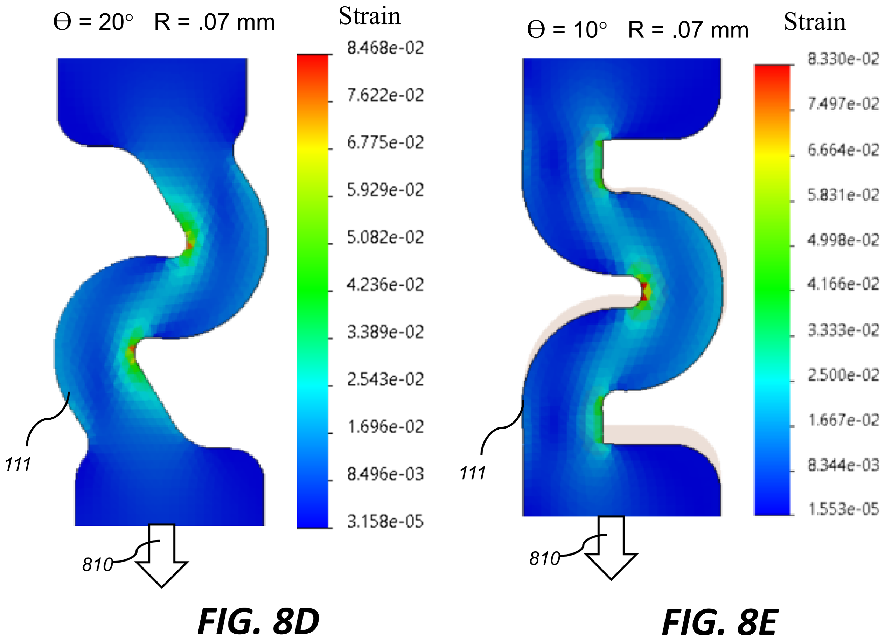

FIG. 8D depicts a substrate 111 having an offset angle Θ of 20 degrees and a radius R of 0.07 mm. In the exemplary configuration, when elongated by 0.1mm, the substrate experiences a maximum strain of 0.08468 or about 8.47%.

FIG. 8E depicts a substrate 111 having an offset angle Θ of 10 degrees and a radius R of 0.07 mm. In the exemplary configuration, when elongated by 0.1mm, the substrate experiences a maximum strain of 0.0833 or about 8.33%.

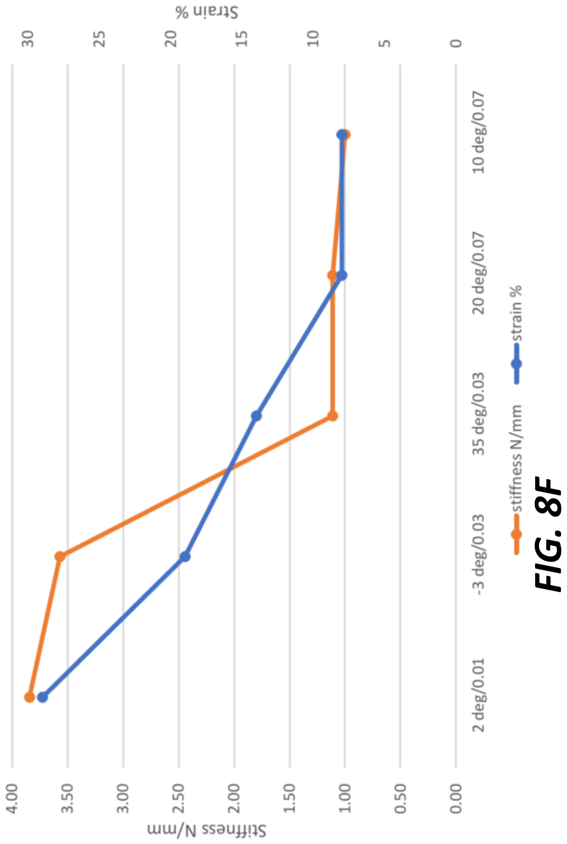

FIG. 8F is a graph depicting stiffness and strain values for each exemplary configuration of FIGS. 8A-8E. In some examples, an offset angle Θ of about 10 degrees to 20 degrees and a radius R of about 0.07 mm provides for reduced stiffness and strain, which may be desirable to provide a flexible substrate for the flexible circuit.

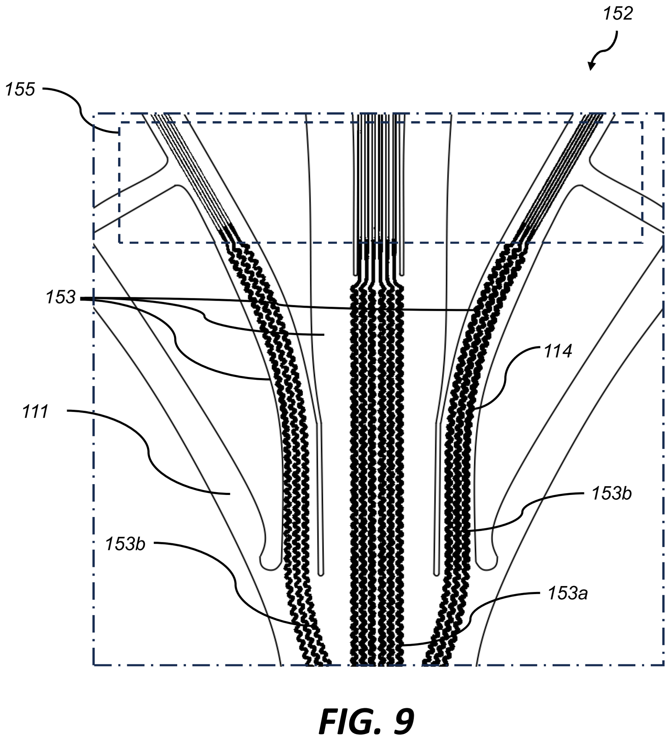

FIG. 9 depicts a proximal portion 152 of a flexible circuit extending distally from a proximal end of the flexible circuit toward a first set of electrodes of a plurality of electrodes (e.g., proximal portion 152 extending from proximal end 102 of the flexible circuit 110 toward a first set of electrodes 112a of a plurality of electrodes 112 as depicted in FIG. 4). In some examples, the substrate 111 at the proximal end 152 of the flexible circuit supports a plurality of electrical traces 114. The electrical traces 114 may each comprise a plurality of bends such that each electrical trace 114 comprises a serpentine shape. The serpentine shape may be utilized to further reinforce the proximal end 152 of the flexible circuit. The serpentine shape may be utilized to provide additional flex at the proximal end 152 of the flexible circuit.

In some examples, the proximal portion 152 of the substrate 111 comprises at least three arms 153 extending from the proximal end of the flexible circuit to the first set of electrodes. In some examples, a center arm 153a of the at least three arms 153 supports at least six electrical traces 114, and each outer arm 153b of the at least three arms 153 supports half the number of electrical traces 114 of the center arm 153a. In some examples, the electrical traces comprise a serpentine shape near the proximal end of the flexible circuit and straighten out at a distal end 155 of the proximal portion 152 of the substrate 111, before reaching the first set of electrodes.

The disclosed technology described herein can be further understood according to the following clauses:
Clause 1: A flexible circuit of an end effector of a medical probe, the end effector extending along a longitudinal axis, the flexible circuit comprising: a first substrate supporting a first electrical trace extending along a portion of the end effector, the first electrical trace comprising a plurality of bends such that the first electrical trace defines a serpentine shape; and a second substrate layered with the first substrate in a thickness direction and supporting a second electrical trace extending along the portion of the end effector, the second electrical trace comprising a plurality of bends such that the second electrical trace defines a serpentine shape, the second electrical trace being offset from the first electrical trace at one or more bends of the plurality of bends.
Clause 2: The flexible circuit of Clause 1 , wherein the first substrate comprises a width equal to a width of the second substrate along the portion of the end effector.
Clause 3: The flexible circuit of Clause 2, wherein the first electrical trace is disposed at a first distance from an edge of the first substrate, and the second electrical trace is disposed at a second distance from a corresponding edge of the second substrate, the first distance being less than or greater than the second distance.
Clause 4: The flexible circuit of any one of Clauses 1 to 3, wherein the second electrical trace is offset from the first electrical trace at a distal corner of the flexible circuit.
Clause 5: The flexible circuit of any one of Clauses 1 to 4, wherein the first substrate comprises at least two electrodes.
Clause 6: The flexible circuit of Clause 5, wherein the first electrical trace extends between and couples the at least two electrodes to each other.
Clause 7: The flexible circuit of Clause 6, wherein the second electrical trace extends between and couples the at least two electrodes to each other.
Clause 8: A flexible circuit of an end effector of a medical probe, the end effector extending along a longitudinal axis, the flexible circuit comprising a substrate supporting an electrical trace extending along a portion of the end effector, the substrate comprising a plurality of bends such that the substrate defines a serpentine shape, the plurality of bends of the substrate being offset relative to the longitudinal axis by an offset angle, and the electrical trace spaced a predetermined distance from an inner radius of each bend.
Clause 9: The flexible circuit of Clause 8, wherein the offset angle is approximately 10 degrees to 35 degrees.
Clause 10: The flexible circuit of Clause 8, wherein the offset angle is approximately 10 degrees to 20 degrees.
Clause 11: The flexible circuit of any one of Clauses 8 to 10, wherein the plurality of bends comprises two bends along the portion of the end effector extending between two electrodes.
Clause 12: The flexible circuit of Clause 11, wherein the two bends are provided between electrodes at a middle portion of the flexible circuit.
Clause 13: The flexible circuit of any one of Clauses 8 to 12, wherein the inner radius of each bend is at least approximately 0.03 millimeters.
Clause 14: The flexible circuit of any one of Clauses 8 to 13, wherein the inner radius of each bend is from approximately 0.03 millimeters to approximately 0.07 millimeters.
Clause 15: The flexible circuit of any one of Clauses 8 to 14, the flexible circuit comprises a plurality of electrical traces and the predetermined distance is measured between an innermost edge of the substrate at the inner radius of each bend and an innermost electrical trace closest to said innermost edge.
Clause 16: The flexible circuit of claim 15, wherein the predetermined distance between the inner radius of each bend and the innermost electrical trace is at least 3 times a radius of the innermost electrical trace at each bend.
Clause 17: A flexible circuit of an end effector of a medical probe, the end effector extending along a longitudinal axis, the flexible circuit comprising: a substrate comprising a proximal portion extending distally from a proximal end of the flexible circuit toward a first set of electrodes of a plurality of electrodes, the substrate configured to support a first electrical trace comprising a plurality of bends such that the electrical trace comprises a serpentine shape; the substrate comprising a middle portion extending between the first set of electrodes and a second set of electrodes and comprising a plurality of bends such that the substrate defines a serpentine shape in the middle portion, the bends being offset relative to the longitudinal axis by an offset angle.
Clause 18: The flexible circuit of Clause 17, wherein the proximal portion of the substrate comprises at least three arms extending from the proximal end of the flexible circuit to the first set of electrodes, each arm supporting a plurality of electrical traces.
Clause 19: The flexible circuit of Clause 18, wherein a center arm of the at least three arms supports at least six electrical traces, and each outer arm of the at least three arms supports half the number of electrical traces of the center arm.
Clause 20: The flexible circuit of any one of Clauses 17 to 19, wherein the first electrical trace comprises a straight section at a distal end of the proximal portion of the substrate.
Clause 21: The flexible circuit of any one of Clauses 17 to 20, wherein the middle portion of the substrate is configured to support a second electrical trace, the second electrical trace comprising being spaced a predetermined distance from an inner radius of each bend.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. A flexible circuit of an end effector of a medical probe, the end effector extending along a longitudinal axis, the flexible circuit comprising:
a first substrate supporting a first electrical trace extending along a portion of the end effector, the first electrical trace comprising a plurality of bends such that the first electrical trace defines a serpentine shape; and
a second substrate layered with the first substrate in a thickness direction and supporting a second electrical trace extending along the portion of the end effector, the second electrical trace comprising a plurality of bends such that the second electrical trace defines a serpentine shape, the second electrical trace being offset from the first electrical trace at one or more bends of the plurality of bends.

2. The flexible circuit of claim 1, wherein the first substrate comprises a width equal to a width of the second substrate along the portion of the end effector.

3. The flexible circuit of claim 2, wherein the first electrical trace is disposed at a first distance from an edge of the first substrate, and the second electrical trace is disposed at a second distance from a corresponding edge of the second substrate, the first distance being less than or greater than the second distance.

4. The flexible circuit of claim 1 to 3, wherein the second electrical trace is offset from the first electrical trace at a distal corner of the flexible circuit.

5. The flexible circuit of claim 1 to 4, wherein the first substrate comprises at least two electrodes, optionally wherein the first electrical trace extends between and couples the at least two electrodes to each other, further optionally wherein the second electrical trace extends between and couples the at least two electrodes to each other.

6. A flexible circuit of an end effector of a medical probe, the end effector extending along a longitudinal axis, the flexible circuit comprising:
a substrate supporting an electrical trace extending along a portion of the end effector, the substrate comprising a plurality of bends such that the substrate defines a serpentine shape, the plurality of bends of the substrate being offset relative to the longitudinal axis by an offset angle, and the electrical trace spaced a predetermined distance from an inner radius of each bend.

7. The flexible circuit of claim 6, wherein the offset angle is approximately 10 degrees to 35 degrees, or approximately 10 degrees to 20 degrees.

8. The flexible circuit of claim 6 or claim 7, wherein the plurality of bends comprises two bends along the portion of the end effector extending between two electrodes, optionally wherein the two bends are provided between electrodes at a middle portion of the flexible circuit.

9. The flexible circuit of claim 6 to 8, wherein the inner radius of each bend is at least approximately 0.03 millimeters, or is from approximately 0.01mm to approximately 0.07mm.

10. The flexible circuit of claim 6 to 9, wherein the flexible circuit comprises a plurality of electrical traces and the predetermined distance is measured between an innermost edge of the substrate at the inner radius of each bend and an innermost electrical trace closest to said innermost edge.

11. The flexible circuit of claim 10, wherein the predetermined distance between the inner radius of each bend and the innermost electrical trace is at least 3 times a radius of the innermost electrical trace at each bend.

12. A flexible circuit of an end effector of a medical probe, the end effector extending along a longitudinal axis, the flexible circuit comprising:
a substrate comprising a proximal portion extending distally from a proximal end of the flexible circuit toward a first set of electrodes of a plurality of electrodes, the substrate configured to support a first electrical trace comprising a plurality of bends such that the electrical trace comprises a serpentine shape
the substrate comprising a middle portion extending between the first set of electrodes and a second set of electrodes and comprising a plurality of bends such that the substrate defines a serpentine shape in the middle portion, the bends being offset relative to the longitudinal axis by an offset angle.

13. The flexible circuit of claim 12, wherein the proximal portion of the substrate comprises at least three arms extending from the proximal end of the flexible circuit to the first set of electrodes, each arm supporting a plurality of electrical traces.

14. The flexible circuit of claim 13, wherein a center arm of the at least three arms supports at least six electrical traces, and each outer arm of the at least three arms supports half a number of electrical traces of the center arm.

15. The flexible circuit of claim 12 to 14, wherein the first electrical trace comprises a straight section at a distal end of the proximal portion of the substrate.
